# EUROPEAN PATENT APPLICATION

(11) **EP 4 563 567 A1**
(43) Date of publication of application: **04.06.2025**
(21) Application number: 23846548.8
(22) Date of filing: 26.07.2023
(51) Int. Cl.: C07C 63/307, C07C 57/15, C07C 57/22, C07C 63/24, C07C 65/03, C07D 233/58, C07D 235/08, C07F 1/08, C07F 3/02, C07F 3/06, C07F 5/06, C07F 15/02, C07F 15/06, C08K 3/08, C08L 101/00

(54) **METHOD FOR PRODUCING METAL-ORGANIC STRUCTURE**

(30) Priority: 28.07.2022 JP 2022120380
(71) Applicant: Atomis Inc., Kobe-shi, Hyogo 650-0047 (JP)
(72) Inventor: ASARI Daisuke, Kobe-shi Hyogo 650-0047 (JP); SAEKI Taku, Kobe-shi Hyogo 650-0047 (JP)
(74) Representative: AOMB Polska Sp. z.o.o.
(86) International application number: PCT/JP2023/027321
(87) International publication number: WO 2024/024818

(57) **Abstract**

[Summary] The present invention provides a simpler method for producing high quality Metal-Organic Frameworks.

[Solution] A method for producing a Metal-Organic Framework according to the present invention includes:preparing an agitator equipped with a turbine and a stator surrounding the turbine, preparing a formulation comprising a metal ion donor, a multidentate ligand, and a solvent, andapplying shear forces generated between the turbine and the stator to the formulation by rotating the turbine relative to the stator in the agitator.

## Description

### TECHNICAL FIELD

The present disclosure relates to a method for producing Metal-Organic Frameworks (MOFs).

### BACKGROUND ART

A group of substances called Metal-Organic Framework has been attracting attention in fields such as gas storage and gas separation. The Metal-Organic Framework is a compound having a structure in which metal atoms are crosslinked with each other by an organic ligand, and typically has porosity. Metal-Organic Frameworks with porosity are also called Porous Coordination Polymers (PCPs).

A liquid phase synthesis method such as a solution method, a hydrothermal method, a microwave method, and an ultrasonic method has been typically used as a method for producing the Metal-Organic Framework. A solid phase synthesis method using a mortar, a ball mill or the like has also been used. In recent years, a method of synthesizing Metal-Organic Frameworks using a biaxial mixing apparatus called an extruder has also been reported. In this method, a Metal-Organic Framework is produced by admixing a first reactant containing a specific metal ion donor and a second reactant containing a specific organic ligand under conditions of prolonged and sustained pressure and shear sufficient to synthesize the Metal-Organic Framework (Patent Literature 1).

The Applicant has disclosed a method for producing a Metal-Organic Framework including simultaneously and continuously applying a centrifugal force and a shear force to a formulation containing a metal ion donor, a multidentate ligand, and a solvent (Patent Literature 2). This document mainly describes an embodiment in which a swirling thin-film type high-speed agitator (see Non-Patent Literature 1) is applied to the production of Metal-Organic Frameworks. The present invention makes it possible to produce a high quality Metal-Organic Framework in a short period of time.

### Citation List

### Patent Literature

[Patent Literature 1] US 9,815,222 B2
Patent Literature 2] WO2019/026872

### Non-Patent Literature

[Non-Patent Literature 1] Haruo Shibuya, "Media-less Dispersion Machines", J. Jpn. Soc. Colour Mater., 86 [7], 265-269 (2013)

### SUMMARY OF THE INVENTION

### Technical Problem

However, the present inventors found that when the above-mentioned method is adopted, it is necessary to use an apparatus having a complicated configuration, and it may be difficult to scale up the method. It is therefore an object of the present invention to provide a method for producing high-quality Metal-Organic Frameworks in a short period of time.

### Solution to Problem

The present inventors have conducted diligent studies in order to solve the above problems. As a result, the present inventors have found a new means of applying a turbine-stator type high-speed stirrer to substance synthesis instead of a swirling thin-film type high-speed agitator, The turbine-stator type high-speed agitator is a type of media-less disperser that has been used mainly for dispersing and/or atomizing particles and droplets (see Non-Patent Literature 1).

Some aspects of the present invention are as described below.
[1] A method for producing a Metal-Organic Framework, comprising:preparing an agitator equipped with a turbine and a stator surrounding the turbine;preparing a formulation comprising a metal ion donor, a multidentate ligand, and a solvent; andapplying shear forces generated between the turbine and the stator to the formulation by rotating the turbine relative to the stator in the agitator.
[2] The method according to [1], wherein the solvent is a poor solvent for at least one of the metal ion donor and the multidentate ligand.
[3] The method according to [1] or [2], wherein the solvent has a Hildebrand solubility parameter of 9.0 or more.
[4] The method according to any one of [1] to [3], wherein an amount of the solvent is in a range of 30 to 2000% by weight based on a total amount of the metal ion donor and the multidentate ligand.
[5] The method according to any one of [1] to [4], wherein the method is carried out at a temperature lower than a normal boiling point of the solvent.
[6] The method according to cany one of [1] to [5], wherein the shortest distance between the turbine and the stator is in the range of 0.1 mm to 5 mm.
[7] The method according to any one of [1] to [6], wherein the Metal-Organic Framework is a Porous Coordination Polymer.

### Advantageous Effects of Invention

The present invention makes it possible to produce a high quality Metal-Organic Framework by a simpler method.

### BRIEF DESCRIPTION OF DRAWINGS

FIG. 1A is a perspective view showing an example of an agitator (homomixer) according to one embodiment of the present invention.
FIG. 1B is a bottom view of the agitator shown in FIG. 1A.
FIG. 2 is a cross-sectional view showing an example of use of the agitator shown in FIGS. 1A and 1B.
FIG. 3A is an exploded perspective view showing an example of an agitator (comb-tooth turbine stator) according to another embodiment of the present invention.
FIG. 3B is a bottom perspective view of the agitator shown in FIG. 3A in an assembled state.
FIG. 4 is a cross-sectional view showing an example of use of the agitator shown in FIGS. 3A and 3B. In FIG. 4, the turbine and stator parts are illustrated as a side view for the sake of simplification in the description.
FIG. 5A shows an XRD (X-ray diffraction) pattern of the Metal-Organic Framework (HKUST-1) synthesized under the conditions of Example 1.
FIG. 5B shows an XRD pattern of the Metal-Organic Framework (HKUST-1) synthesized under the conditions of Comparative Example A2.
FIG. 6A shows an XRD pattern of the Metal-Organic Framework [MOF-74(Mg)] synthesized under the conditions of Example 7.
FIG. 6B shows an XRD pattern of the Metal-Organic Framework [MOF-74(Mg)] synthesized under the conditions of Comparative Example A4.
FIG. 7A shows the XRD pattern of the Metal-Organic Framework (ZIF-8) synthesized under the conditions of Example 12.
FIG. 7B shows the XRD pattern of the Metal-Organic Framework (ZIF-8) synthesized under the conditions of Comparative Example A13.
FIG. 8A shows the XRD pattern of the Metal-Organic Framework (ZIF-67) synthesized under the conditions of Example 26.
FIG. 8B shows the XRD pattern of the Metal-Organic Framework (ZIF-67) synthesized under the conditions of Comparative Example A32.

### DESCRIPTION OF EMBODIMENTS

The production methods according to an embodiment of the present invention will hereinafter be described. When referring to the drawings, the same reference numerals are given to the components exhibiting the same or similar functions, and duplicate description will be omitted.

A method for producing a metal organic framework according to an embodiment of the invention comprises: preparing an agitator including a turbine and a stator surrounding the turbine; preparing a blend containing a metal ion donor, a multidentate ligand, and a solvent; and applying a shear force generated between the turbine and the stator to the blend in the agitator by rotating the turbine relative to the stator. The method may include introducing a separately prepared formulation into a reaction vessel. Alternatively, this method may include forming the above formulation inside a reaction vessel by sequentially adding the components of the formulation into the reaction vessel.

The agitator may be a high speed agitator known as a turbine/stator type agitator. The agitator includes a turbine and a stator surrounding the turbine. In addition, in this agitator, the turbine is configured to rotate relative to the stator. This enables the application of a stronger shear force, generated between the turbine and the stator, to the formulation serving as the reactant.

As the turbine/stator type agitator, for example, a homomixer or a comb-tooth type turbine-stator can be used. The above-mentioned agitator may be an in-line disperser or a hybrid agitator that includes a turbine/stator-type agitator. The use of these agitators as conventional media-less dispersers is described in detail, for example, in Non-Patent Literature 1. Examples of the configuration of the homomixer and the comb-tooth turbine stator will be described below with reference to the drawings.

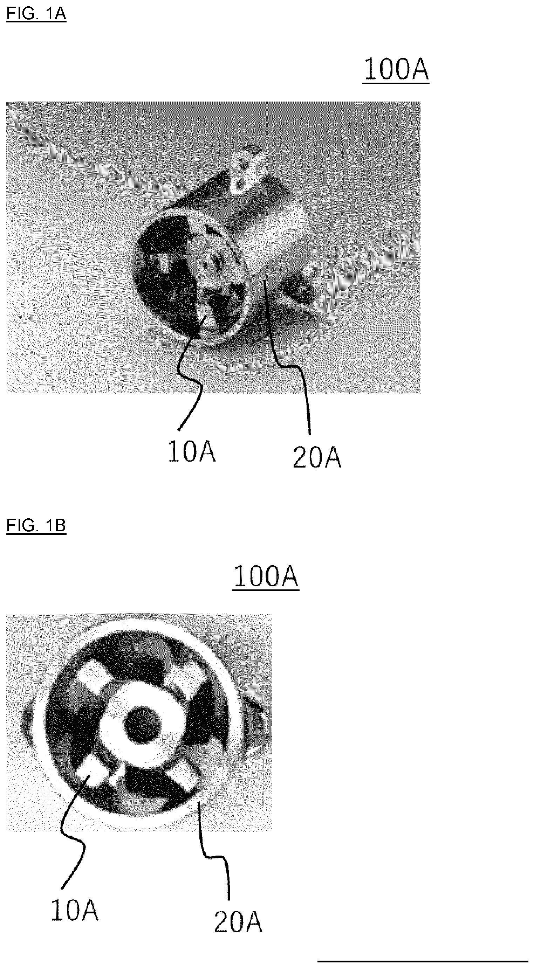

FIG. 1A is a perspective view showing an example of an agitator according to one embodiment of the present invention. FIG. 1B is a bottom view of the agitator shown in FIG. 1A. The agitator 100A illustrated in FIGS. 1A and 1B is a homomixer, and includes a turbine 10A and a stator 20A surrounding the turbine 10A. In the agitator 100A, the turbine 10A is configured to be rotatable relative to the stator 20A.

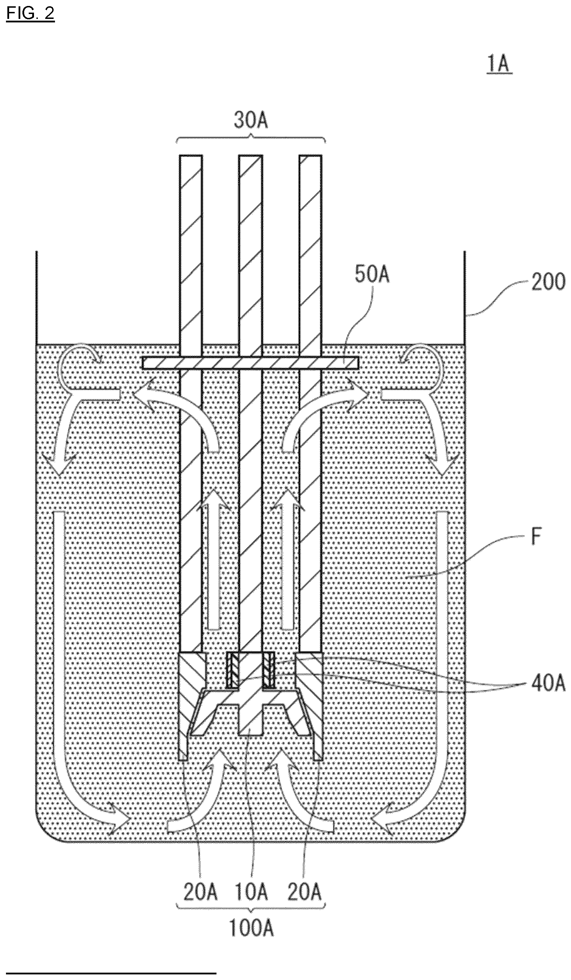

FIG. 2 is a cross-sectional view showing an example of use of the agitator shown in FIGS. 1A and 1B. The reaction apparatus 1A shown in FIG. 2 includes an agitator 100A and a reaction vessel 200. The agitator 100A includes a turbine 10A and a stator 20A. Turbine 10A and stator 20A are optionally connected to a shaft 30A via a bearing 40A. A deflector plate 50A is optionally provided above shaft 30A. The reaction apparatus 1A can have the same configuration as when the agitator 100A is used as a media-less disperser, except for differences in the formulation F, which will be described later.

The turbine 10A, stator 20A, shaft 30A, and deflector plate 50A are typically made from an alloy such as stainless steel. The bearing 40A may be made of, for example, a plastic material such as Teflon (registered trademark) or an alloy such as stainless steel. In the former case, replacement is easy when worn out. In the latter case, the possibility of contamination of the reaction system can be reduced.

When the Metal-Organic Framework is produced using the reaction apparatus 1A, first, the formulation F is introduced into the reaction vessel 200. Next, in the agitator 100A, the turbine 10A is rotated at high speed relative to the stator 20A. In this way, as exemplarily illustrated by the hollow arrows in FIG. 2, the formulation F is drawn in from the lower part of the stator 20A, ejected upward, collides with the deflection plate 50A, and circulates within the reaction vessel 200. At this time, a strong shear force is applied to the formulation F between the turbine 10A and the stator 20A. By applying such a shear force, the desired Metal-Organic Framework can be synthesized from Formulation F.

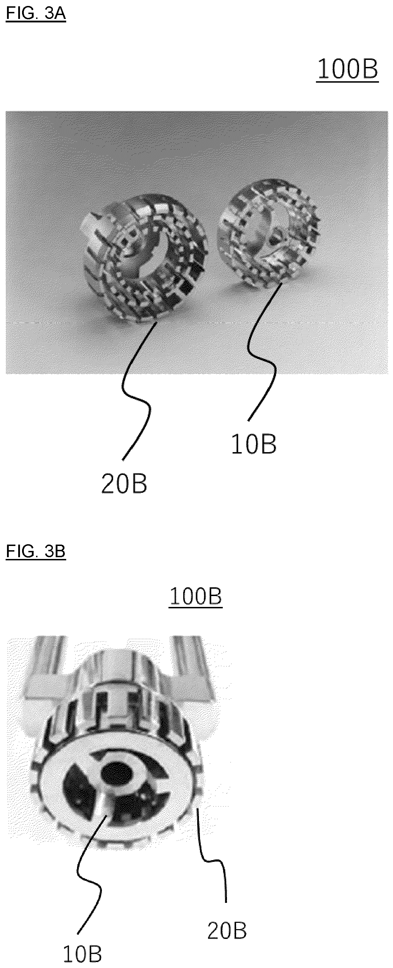

FIG. 3A is an exploded perspective view showing an example of an agitator according to another embodiment of the present invention. FIG. 3B is a bottom perspective view of the agitator shown in FIG. 3A in an assembled state. The agitator 100B illustrated in FIGS. 3A and 3B is a comb-tooth turbine stator, and includes a turbine 10B and a stator 20B surrounding the turbine 10B. In the agitator 100B, the turbine 10B is configured to be rotatable relative to the stator 20B.

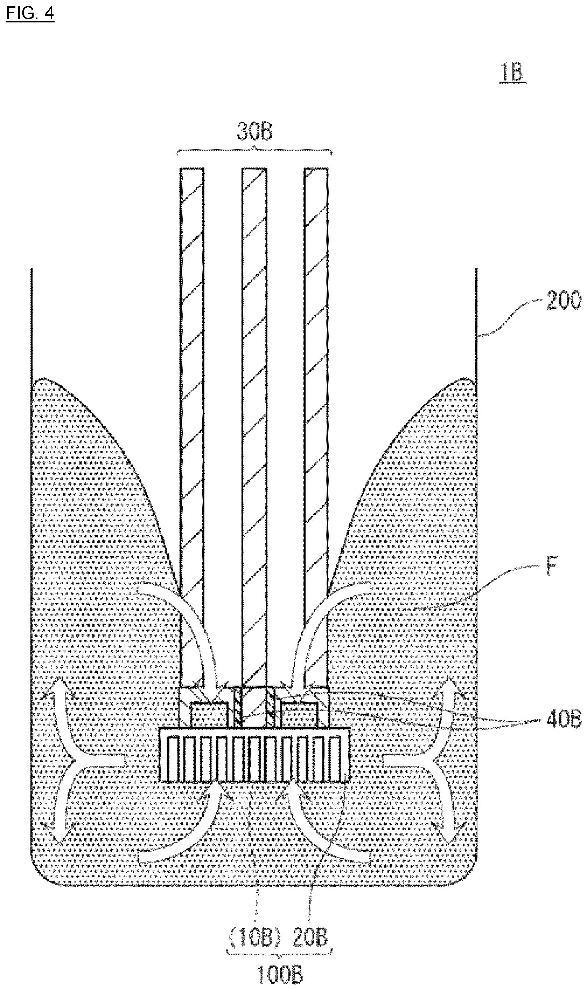

FIG. 4 is a cross-sectional view (partially a side view) showing an example of the use of the agitator illustrated in FIGS. 3A and 3B. The reaction apparatus 1B shown in FIG. 4 includes an agitator 100B and a reaction vessel 200. The agitator 100B includes a turbine 10B (not shown) and a stator 20B. Turbine 10B and stator 20B are optionally connected to a shaft 30B via a bearing 40B. The reaction apparatus 1B can have the same configuration as when the agitator 100B is used as a media-less disperser, except for differences in the formulation F, which will be described later. The materials for the turbine 10B, the stator 20B, the shaft 30B, and the bearings 40B may be, for example, the same as those described above for the turbine 10A, the stator 20A, the shaft 30A, and the bearings 40A.

When the Metal-Organic Framework is produced using the reaction apparatus 1B, first, the formulation F is introduced into the reaction vessel 200. Next, in the agitator 100B, the turbine 10B is rotated at high speed relative to the stator 20B. In this way, as exemplarily illustrated by the hollow arrows in FIG. 4, the formulation F is drawn in from both the upper and lower parts of the stator 20B, ejected laterally from the comb-tooth-shaped slits, collides with the wall of the reaction vessel 200, and circulates within the reaction vessel 200. At this time, a strong shear force is applied to the formulation F between the turbine 10B and the stator 20B. By applying such a shear force, the desired Metal-Organic Framework can be synthesized from Formulation F.

The inventors of the present invention have found that using a turbine/stator-type agitator as described above enables the production of Metal-Organic Frameworks of higher quality compared to using high-speed stirring blades such as disk turbines or dispersers, or conventional stirrers. This difference is presumed to be mainly due to the large shear force generated between the turbine and the stator.

Furthermore, the inventors of the present invention have also found that using the above-mentioned agitator facilitates scale-up compared to using high-speed stirring blades or stirrers. When using high-speed stirring blades or stirrers, scaling up (i.e., increasing the size of the reaction vessel) increases the distance between the vessel and the stirring blades or stirrer. This makes it difficult to apply shear force to the reactants, resulting in a decrease in the amount and/or quality of the obtained Metal-Organic Framework. In contrast, when using the above-mentioned agitator, scaling up does not change the distance between the turbine and the stator (hereinafter referred to as 'clearance'). Therefore, when using the above-mentioned agitator, it becomes possible to produce high-quality Metal-Organic Frameworks with high yield, even when scaling up.

Additionally, when using a swirling thin-film type high-speed agitator, it is possible to synthesize high-quality Metal-Organic Frameworks in a short reaction time, as disclosed in Patent Literature 2. However, this agitator has a complicated structure and is not very versatile. In particular, in this agitator, the shear force is generated by bringing the reactants into contact with the inner wall of the reaction vessel mainly by centrifugal force generated by the rotating blades. Therefore, when attempting to scale up using this apparatus, it is necessary to enlarge the reaction vessel and accordingly increase the size of the rotor blades. Therefore, in a synthesis using this agitator it is difficult to scale up the synthesis by simply increasing the size of the reaction vessel. In contrast, when using the above-mentioned turbine/stator-type agitator, scaling up can be easily achieved by applying the same agitator to a larger reaction vessel.

Patent Literature 2 also describes an embodiment in which a swirling thin-film type high-speed agitator is used in a continuous manner. In this case, it is easier to scale up compared to using this agitator in a batch system. However, if the reaction is carried out in a continuous mode, the viscosity of Formulation F must be sufficiently low. That is, in this case, the amount of solvent in Formulation F needs to be increased. In contrast, embodiments of the present invention that utilize turbine/stator type agitators have no such limitations. By keeping the amount of the solvent low, it is possible to reduce the production costs of the Metal-Organic Framework and the environmental load caused by the synthesis.

In the above turbine/stator type agitator, the shortest distance (clearance) between the turbine and the stator is, for example, in the range of 0.1 mm to 5 mm, and preferably in the range of 0.3 mm to 1.5 mm. If the clearance is too small, the reactants will tend to become stuck between the turbine and the stator. If the clearance is too large, the shear force applied to the reactants is reduced, making it difficult to synthesize the Metal-Organic Framework.

Usable turbine/stator type agitators include, for example, those shown in Table 1 below. These turbine/stator type agitators are commercially available for dispersing and/or atomizing particles and liquid droplets. It should be noted that these are merely examples, and other devices may be used as long as they are an agitator including a turbine and a stator.

**Table 1**

| Manufacturer | Equipment Name | Model Number |
|---|---|---|
| Primix Co., Ltd. | Homomixer MARK II | |
| | Neo Kaiser | |
| | Neo Mixer | |
| | Pipeline Homomixer | |
| | Homomic Line Mill | |
| | Homomic Line Flow | |
| Mizubo Industry Co., Ltd. | Desktop Quick Homomixer | LR Model |
| | Test Vacuum Emulsification Device | APVQ Model |
| | Test Vacuum Emulsification Device | PVQ Model |
| | Lift-type Stirring Device | SLR |
| | Vacuum Dissolution Tank | VD Model |
| | Mobile Dissolution Tank | TD Model |
| | Raw Material Dissolution Tank | D Model |
| | 3-axis / 4-axis Vacuum Kneading Device | VKD Model |
| | Bottom Circulation Mixer | TW Model |
| | Pipeline Mixer | PM Model |
| | Upper and Lower Triple Stirring Type | VTU Model |
| | Upper Triple Stirring Type | VT, FVT Model |
| | Upper Double Stirring Type | VQ, OQ |
| | Upper Independent 2-axis / 3-axis Type | VII, FOII, VIII, FOIII |
| SILVERSON | Batch Mixers | |
| | High Shear In-Line Mixers | LS Series |
| | Silverson Mixer Homogenizers | |
| | Flashmix Powder/Liquid Mixers | FMX Series |
| | Flashblend Powder/Liquid Mixing Systems | FLB Series |
| | Bottom Entry Mixers | BE Series |
| | Disintegrator/ Dissolvers | |
| | High Viscosity In-Line Mixers | |
| | Pilot Scale Batch Mixers | AX5 Series |
| | Laboratory Mixers | L5 Series |
| | Verso Laboratory In-Line Mixers | |
| | Laboratory Scale Powder/Liquid Mixer | FMX5 |
| Chuo Rika Co., Ltd. | High-Speed Homomixer | LZB14-HM-1 |
| | | LZB33-HM-2 |
| | | LZB-46-HM-3 |
| KINEMATICA | POLYTRON | |
| | MEGATRON | |
| | REACTRON | |
| | BIOTRONA | |
| Microtech Nichion Co., Ltd. | Hiscotron | |
| IKA | ULTRA-TURRAX | |
| OMNI international | Omni Mixer / Macro | |
| | General Laboratory Homogenizer | GLH850 |
| | Omni Micro | |
| | TH Tissue Homogenizer | |
| | Tissue Master | |
| | Omni THq Homogenizer | |
| MXBAOHENG | High Speed Homogenizer Adjustable | |
| As One Co., Ltd. | Homomixer | HM-300 |
| | | HM-312 |

There are no particular restrictions on the type of Metal-Organic Frameworks (MOFs) to be produced. Appropriately combining the type and coordination number of the metal ion with the type and topology of the multidentate ligand leads to a MOF with a desired structure. The MOF may contain two or more types of metal elements, and may contain two or more types of multidentate ligands. The MOF may further contain monodentate ligand(s). The MOF may be porous. In other words, the MOF may be a Porous Coordination Polymer (PCP).

Specific examples of the MOF include those listed in the literatures below:
Reference 1: Yabing He et al., Methane Storage in Metal-Organic Frameworks, Chem Soc Rev, 2014
Reference 2: Jarad A. Mason et al., Evaluating Metal-Organic Frameworks for natural gas storage, Chem. Sci., 2014, 5, 32-51
Reference 3: WO2019/026872

As described above, the formulation used as a raw material for the MOF contains a metal ion donor, a multidentate ligand, and a solvent. As the metal ion donor and the multidentate ligand, any substance can be used as long as it is suitable as a combination for synthesizing a MOF.

The metal elements of the metal ion donor can be, for example, any elements belonging to alkali metals (Group 1), alkaline earth metals (Group 2), or transition metals (Groups 3 to 12). The metal element is typically selected from the group consisting of magnesium, calcium, iron, aluminum, zinc, copper, nickel, cobalt, zirconium, and chromium. The metal ion donor may contain a plurality of metal elements. Alternatively, a plurality of metal ion donors containing different metal elements may be used in combination.

As the metal ion donor, a metal salt is typically used. The metal ion donor may be an organic salt or an inorganic salt. The metal ion donor is typically selected from the group consisting of hydroxides, carbonates, acetates, sulfates, nitrates, and chlorides. A plurality of metal ion donors containing the same metal element may be used in combination. The metal ion donor may be in the form of a so-called secondary building unit (SBU). Such secondary building units can be chosen from any of those used to synthesize known metal organic frameworks.

The multidentate ligand is typically an organic multidentate ligand and is selected from the group consisting of, for example, carboxylic acid anions, amine compounds, sulfonic acid anions, phosphate anions, and heterocyclic compounds. Examples of the carboxylic acid anion include dicarboxylic acid anion and tricarboxylic acid anion. Specific examples include anions of citric acid, malic acid, terephthalic acid, isophthalic acid, trimesic acid, and derivatives thereof. Examples of the heterocyclic compound include bipyridine, imidazole, adenine, and derivatives thereof. A plurality of multidentate ligands may be used.

The type of solvent contained in the above formulation is not particularly limited, and a solvent generally used for synthesizing a MOF can be used. However, the solvent may preferably be a poor solvent for at least one of the metal ion donor and the multidentate ligand. With such a configuration, the formulation does not become a complete solution but becomes a semi-solid, typically a slurry, with solids remaining. This makes it possible to more effectively apply shear forces to the above-mentioned formulation. Here, the term "poor solvent" for an object means that the solubility in a solvent of the object is 1g/50mL (=20 g/L) or less at 25 °C and at atmospheric pressure. Examples of solvents that can be used include water, alcohols such as methanol, ethanol and isopropyl alcohol, carboxylic acids such as formic acid and acetic acid, amides such as N, N-dimethylformamide (DMF) and N, N-diethylformamide (DEF), and esters such as ethyl acetate. A mixture of a plurality of solvents may also be used.

It is particularly preferable that the above solvent has a Hildebrand solubility parameter of 9.0 or more. In this case, it becomes possible to synthesize a Metal-Organic Framework of higher quality. Table 2 shows examples of Hildebrand solubility parameters for representative solvents. When a mixed solvent is used, the Hildebrand solubility parameter is a weighted average value according to the mixing ratio. As shown in Table 2, specific examples of solvents having a Hildebrand solubility parameter of 9.0 or greater include tetrahydrofuran, chloroform, acetone, methylene chloride, ethylene dichloride, dioxane, isopropyl alcohol, ethanol, dimethylformamide, acetonitrile, acetic acid, dimethyl sulfoxide, methanol, ethylene glycol, and water.

**Table 2**

| Solvent Name | Solubility Parameter δ |
|---|---|
| Isopropyl Ether | 7.0 |
| n-Hexane | 7.3 |
| Cyclohexane | 8.2 |
| Carbon Tetrachloride | 8.6 |
| Ethyl Acetate | 8.6 |
| Toluene | 8.9 |
| Tetrahydrofuran | 9.1 |
| Chloroform | 9.3 |
| Acetone | 9.4 |
| Methylene Chloride | 9.6 |
| Dichloroethene | 9.7 |
| Dioxane | 9.8 |
| Isopropyl Alcohol | 10.2 |
| Ethanol | 11.2 |
| Dimethylformamide | 11.5 |
| Acetonitrile | 11.8 |
| Acetic Acid | 12.4 |
| Dimethyl Sulfoxide | 12.8 |
| Methanol | 12.9 |
| Ethylene Glycol | 14.7 |
| Water | 21.0 |

| | |
|---|---|
| δ: Hildebrand Solubility Parameter | |

The amount of the solvent based on the total amount of the metal ion donor and the multidentate ligand is, for example, in the range of 25 to 2000% by weight, preferably in the range of 25 to 1000% by weight. Adopting such a configuration makes it possible to improve the production efficiency of the MOF. Generally, embodiments of the invention that use turbine/stator type agitators allow for lower amounts of solvent (i.e., more concentrated formulations) than those that use swirling thin-film type high-speed agitators. For example, in some embodiments of the present invention, even when the amount of the solvent is 100% by weight or less (for example, 25 to 100% by weight), a high-quality Metal-Organic Framework can be synthesized. By keeping the amount of the solvent low, it is possible to reduce the production costs of the Metal-Organic Framework and the environmental load caused by the synthesis.

The above formulation may further contain additional substances such as reaction accelerators. The reaction accelerator is, for example, a basic substance or an acidic substance, and is typically a basic substance. Examples of the basic substance include diethylamine, triethylamine, 2,6-lutidine, pyridine, imidazole, potassium hydroxide, and sodium hydroxide. Examples of acidic substances include formic acid, acetic acid, trifluoroacetic acid, sulfuric acid, nitric acid, hydrochloric acid, and phosphoric acid. As an additional substance, a plurality of reaction accelerators may be used in combination. As an additional substance, a reaction control agent may also be added.

The above production is preferably carried out while controlling the reaction temperature. In that case, the mixing is preferably carried out at a temperature lower than the normal boiling point of the solvent. The mixing is carried out, for example, at a temperature of 80°C or lower, preferably 60°C or lower. In this way, it is possible to produce MOF in a state in which the solvent in the formulation remains in appropriate amount.

In the above production, the rotation speed of the agitator (ie, the rotation speed of the turbine) is, for example, 1,000 to 20,000 rpm, and preferably 5,000 to 15,000 rpm. The rotation speed can be appropriately adjusted depending on the concentration of the formulation, etc. If the rotational speed is excessively low, the shear force applied to the formulation as the reactant may be insufficient. If the rotation speed is too high, it may become difficult to control the temperature of the reaction system.

### EXAMPLES

In order to compare the use of a turbine/stator type high-speed agitator with the use of a non-turbine/stator type high-speed agitator, the following demonstration experiment was carried out.

### Examples 1 to 30: Turbine/stator type high-speed agitator

The metal ion donor, polydentate ligand, solvent, and optionally reaction promoter shown in Table 3 were placed in a batch reaction vessel. Next, high-speed agitation was performed using a turbine/stator-type high-speed agitator [Homomixer MARK II 2.5 type: Primix Corporation, or Neokaiser (Registered Trademark): Primix Corporation] under the reaction conditions shown in Table 3. The MOFs were thereby obtained. Note: Neokaiser is a type of comb-tooth turbine and stator, as described with reference to FIG. 4.

### Comparative Examples A1 to A33: High-speed stirring blade or stirrer

The metal ion donor, polydentate ligand, solvent, and optionally reaction promoter shown in Table 4 were placed in a batch reaction vessel. Next, high-speed agitation was performed using a high-speed stirring blade (Homodisper: Primix Corporation) or a commercially available stirrer under the reaction conditions shown in Table 4.

### Comparative Examples B1 to B39: Swirling Thin Film Type High Speed Agitator

The metal ion donor, multidentate ligand, solvent, and optionally a reaction promoter shown in Table 5 were added to a swirling thin-film type high-speed agitator [Filmix (registered trademark) 56-L type; Primix Corporation] . Next, high-speed agatation was performed under the reaction conditions shown in Table 5.

### Evaluation

The sample obtained by each of the above methods was dried under reduced pressure for 24 hours at room temperature using a vacuum desiccator (MVD-300; AS ONE Corporation). The dried sample was subjected to XRD measurement using an X-ray diffractometer (MiniFlex; Rigaku Co., Ltd.). Further, at least some of the samples were heated and vacuum-dried at 140°C for 4 hours using a gas adsorption pretreatment apparatus (BERPREP-vacIII; MicrotracBEL), and then the BET specific surface area (N₂; 77K) was measured using a gas adsorption apparatus (BELSORP-miniX; MicrotracBEL).

The quality of the obtained Metal-Organic Framework was evaluated by the presence or absence of a crystalline peak by XRD measurement and the size of the BET specific surface area S_{BET}. These results are summarized in Tables 3 to 5.

The following abbreviations were used in Tables 3 to 5.
BTC: 1,3,5-benzenetricarboxylic acid (trimesic acid)
2,5-DHTP: dihydroxyterephthalic acid
2-Mim or Mim: 2-methylimidazole
1,3-BDC or iBDC: isophthalic acid
DOBPDC: 4,4'-dioxidobiphenyl-3,3'-dicarboxylic acid
Im: Imidazole
BIm: benzimidazole
1,4-BDC or pBDC: terephthalic acid
IPA: isopropyl alcohol
AcOH: acetic acid
TEA: triethylamine
DEA: diethanolamine
INA: 4-pyridinecarboxylic acid
ADC: acetylenedicarboxylic acid
DOT: dihydroxyterephthalic acid
BTC3Na: trisodium 1,3,5-benzenetricarboxylate

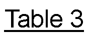

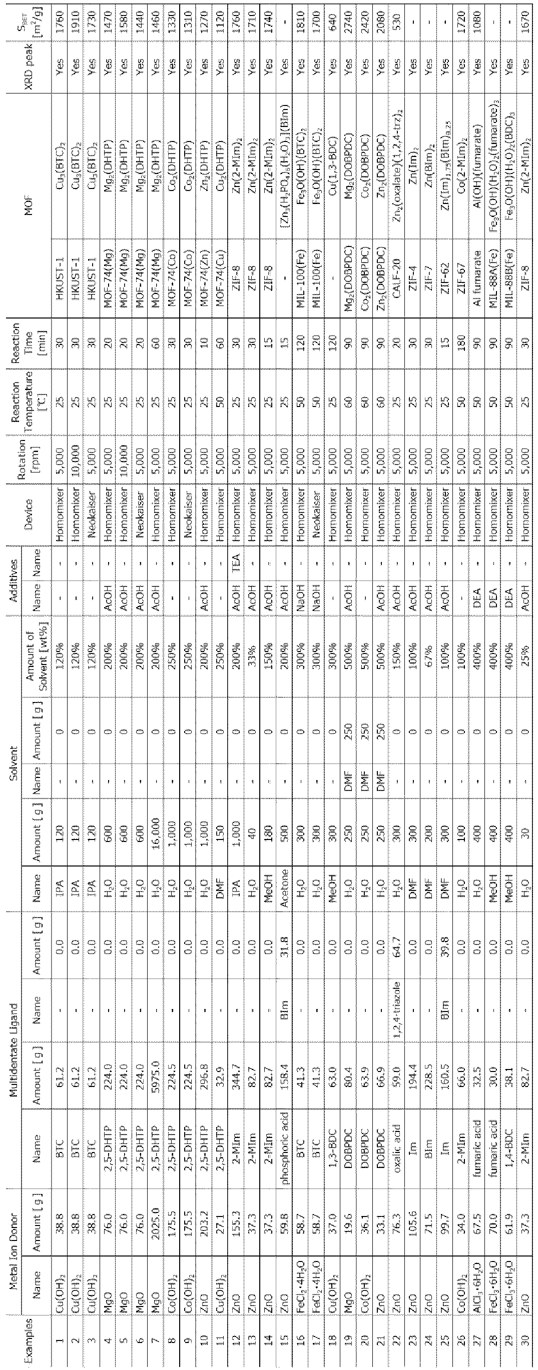

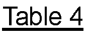

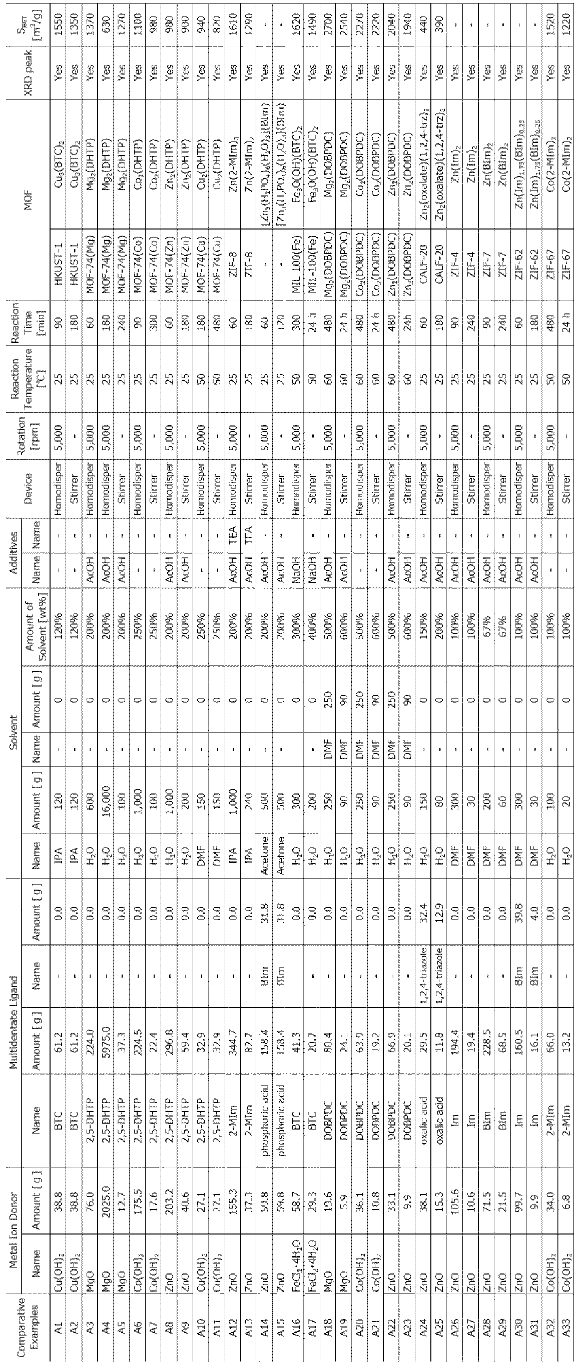

**Table 5**

| Comparative Examples | Metal Ion Donor | | Multidentate Ligand | | Solvent | | | Gas Replacement | Linear Velocity [m/s] | Reaction Temperature [°C] | **Reaction Time** [min] | MOF | XRD peak | S_{BET} [m²/g] |
|---|---|---|---|---|---|---|---|---|---|---|---|---|---|---|
| | Name | Amount [g] | Name | Amount [g] | Name | Amount [g] | Amount of Solvent [wt%] | | | | | | | |
| B1 | Cu(OH)₂ | 8.8 | BTC | 12.6 | EtOH | 42.8 | 200% | None | 30 | 25 | 15 | Cu₃(BTC)₂ | Yes | 1885 |
| B2 | Cu(OH)₂ | 13.2 | BTC | 18.9 | EtOH | 32.1 | 100% | None | 30 | 25 | 15 | Cu₃(BTC)₂ | Yes | *1771* |
| B3 | Cu(OH)₂ | 6.6 | BTC | 9.5 | EtOH | 48.3 | 300% | None | 30 | 25 | 15 | Cu₃(BTC), | Yes | 1877 |
| B4 | Cu(OH)₂ | 2.9 | BTC | 4.2 | EtOH | 71.0 | 1000% | None | 30 | 25 | 15 | Cu₃(BTC)₂ | Yes | 1588 |
| B5 | Cu(OH)₂ | 8.8 | BTC | 12.6 | EtOH | 42.8 | 200% | None | 30 | 60 | 15 | Cu₃(BTC)₂ | Yes | 1822 |
| B6 | Cu(OH)₂ | 8.8 | BTC | 12.6 | EtOH | 42.8 | 200% | None | 30 | 80 | 15 | Cu₃(BTC)₂ | Yes | 1610 |
| B7 | Cu(OH)₂ | 8.8 | BTC | 12.6 | EtOH | 42.8 | 200% | Dry Air | 30 | 25 | 15 | Cu₃(BTC)₂ | Yes | 1885 |
| B8 | Cu(OH)₂ | 8.8 | BTC | 12.6 | MeOH | 42.8 | 200% | None | 30 | 25 | 15 | Cu₃(BTC)₂ | Yes | 1595 |
| B9 | Cu(OH)₂ | 8.8 | BTC | 12.6 | MeOH | 42.8 | 200% | None | 30 | 60 | 15 | Cu₃(BTC)₂ | Yes | 1515 |
| B10 | Cu(OH)₂ | 8.8 | BTC | 12.6 | MeOH | 42.8 | 200% | None | 30 | 80 | 15 | Cu₃(BTC)₂ | Yes | 1309 |
| B11 | Cu(OH)₂ | 8.8 | BTC | 12.6 | DMF | 42.8 | 200% | None | 30 | 25 | 15 | Cu₃(BTC)₂ | Yes | 1863 |
| B12 | Cu(OH)₂ | 10.6 | BTC | 15.1 | DEF | 51.4 | 200% | None | 30 | 25 | 15 | Cu₃(BTC)₂ | Yes | 1763 |
| B13 | Cu(OAc)₂·H₂O | 4.3 | BTC | 12.6 | EtOH | 42.3 | 250% | None | 30 | 25 | 15 | Cu₃(BTC)₂ | Yes | 1768 |
| B14 | Cu(OAc)₂·H₂O | 7.8 | iBDC | 13.3 | DMF | 52.8 | 250% | None | 30 | 25 | 15 | Cu(iBDC) | Yes | 343 |
| B15 | Cu(OH)₂ | 5.9 | iBDC | 13.3 | EtOH | 57.6 | 300% | None | 30 | 25 | 15 | Cu(IBDC) | Yes | 312 |
| B16 | Cu(OH)₂ | 5.9 | INA | 14.8 | EtOAc | 41.4 | 200% | None | 30 | 25 | 15 | Cu(INA), | Yes | - |
| B17 | ZnO | 6.5 | DOT | 15.9 | DMF/H₂O | 67.2 | 300% | None | 30 | 25 | 15 | Zn₂(DOT) | Yes | 1391 |
| B18 | ZnO | 8.1 | MIm | 16.4 | H₂O | 49.0 | 200% | None | 30 | 25 | 15 | Zn(Mim)₂ | Yes | 1690 |
| B19 | 2ZnCO₃·3Zn(OH)₂·H₂O | 28.3 | MIm | 1.6 | H₂O | 59.8 | 200% | None | 30 | 25 | 15 | Zn(Mim)₂ | Yes | 1780 |
| B20 | ZnO | 9.8 | pBDC | 15.0 | DMF (Super Dehydrated) | 49.6 | 200% | None | 30 | 25 | 15 | Zn₄O(BDC)₃ | Yes | 418 |
| B21 | ZnO | 9.8 | pBDC | 15.0 | DMF (Super Dehydrated) | 49.6 | 200% | Dry Air | 30 | 25 | 15 | Zn₄O(BDC)₃ | Yes | 2012 |
| B22 | ZnO | 9.8 | pBDC | 15.0 | DMF (Super Dehydrated) | 49.6 | 200% | Nitrogen | 30 | 25 | 15 | Zn₄O(BDC)₃ | Yes | 1924 |
| B23 | ZnO | 9.8 | pBDC | 15.0 | DMF (Super Dehydrated) | 49.6 | 200% | Argon | 30 | 25 | 15 | Zn₄O(BDC)₃ | Yes | 2101 |
| B24 | 2ZnCO₃·3Zn(OH)₂·H₂O | 22.7 | pBDC | 5.0 | DMF (Super Dehydrated) | 55.4 | 200% | None | 30 | 25 | 15 | Zn₄O(BDC)₃ | Yes | 217 |
| B25 | 2ZnCO₃·3Zn(OH)₂·H₂O | 22.7 | pBDC | 5.0 | DMF (Super Dehydrated) | 55.4 | 200% | Nitorogen | 30 | 25 | 15 | Zn₄O(BDC)₃ | Yes | 1781 |
| B26 | ZrCl₂O·8H₂O | 19.3 | BTC | 4.2 | HCOOH/H₂O | 70.5 | 300% | None | 60 | 25 | 15 | Zr₆O₄(OH)₄(BTC)₂(HCOO)₆ | Yes | - |
| B27 | ZrCl₂O·8H₂O | 16.1 | pBDC | 8.3 | HCOOH/H₂O | 61.0 | 250% | None | 60 | 25 | 15 | Zr₆O₄(OH)₄(pBDC)₆ | Yes | 1356 |
| B28 | Ca(OH), | 8.8 | BTC | 12.6 | EtOH | 42.8 | 200% | None | 30 | 25 | 15 | Ca₃(BTC)₂ | Yes | - |
| B29 | Ca(OH)₂ | 7.8 | pBDC | 13.3 | EtOH | 63.3 | 300% | None | 30 | 25 | 15 | Ca(pBDC) | Yes | - |
| B30 | Ca(OH), | 9.8 | ADC | 11.4 | EtOAc | 63.6 | 300% | None | 30 | 25 | 15 | Ca(ADC) | Yes | - |
| B31 | Mg(OH), | 4.7 | DOT | 15.9 | EtOH | 41.2 | 200% | None | 30 | 25 | 15 | Mg₂(DOT) | Yes | - |
| B32 | y-Al₂O₃ | 12.2 | pBDC | 10.0 | DMF/H₂O | 66.6 | 300% | None | 60 | 25 | 15 | Al(OH)(BDC) | Yes | - |
| B33 | NaAlO₂ | 7.4 | pBDC | 15.0 | DMF/H₂O | 67.2 | 300% | None | 60 | 25 | 15 | Al(OH)(BDC) | Yes | - |
| B34 | γ-Al₂O₃ | 14.3 | Fumarate | 8.1 | H₂O | 67.2 | 300% | None | 60 | 25 | 15 | Al(OH)(Fumalate) | Yes | 1099 |
| B35 | NaAlO₂ | 8.2 | Fumarate | 11.6 | H₂O | 59.4 | 300% | None | 60 | 25 | 15 | Al(OH)(Fumalate) | Yes | 1132 |
| B36 | FeCl₂·4H₂O | 11.9 | BTC3Na | 11.0 | H₂O | 68.7 | 300% | None | 30 | 25 | 15 | Fe₃O(OH)(BTC)₂ | Yes | 1039 |
| B37 | FeCl₂·4H₂O | 11.9 | BTC3Na | 11.0 | H₂O | 68.7 | 300% | Oxygen | 30 | 25 | 15 | Fe₃O(OH)(BTC)₂ | Yes | 1671 |
| B38 | CrCl₂ | 9.8 | pBDC | 13.3 | DMF/H₂O | 57.8 | 250% | Oxygen | 30 | 25 | 15 | Cr₃(OH)(H₂O)₂O(pBDC)₂ | Yes | - |
| B39 | ZnO | 10.6 | Mlm | 21.4 | H₂O | 8.0 | 25% | None | 30 | 25 | 15 | Unable to synthesize | - | - |

A comparison of Table 3 and Table 4 shows that by using a turbine/stator-type high-speed agitator, Metal-Organic Frameworks of higher quality can be synthesized while significantly reducing the reaction time, compared to using high-speed stirring blades or a stirrer. A comparison of Table 3 and Table 5 shows that by using a turbine/stator-type high-speed agitator, it is possible to synthesize Metal-Organic Frameworks of equal or better quality at a larger scale, compared to using a swirling thin-film type high-speed agitator.

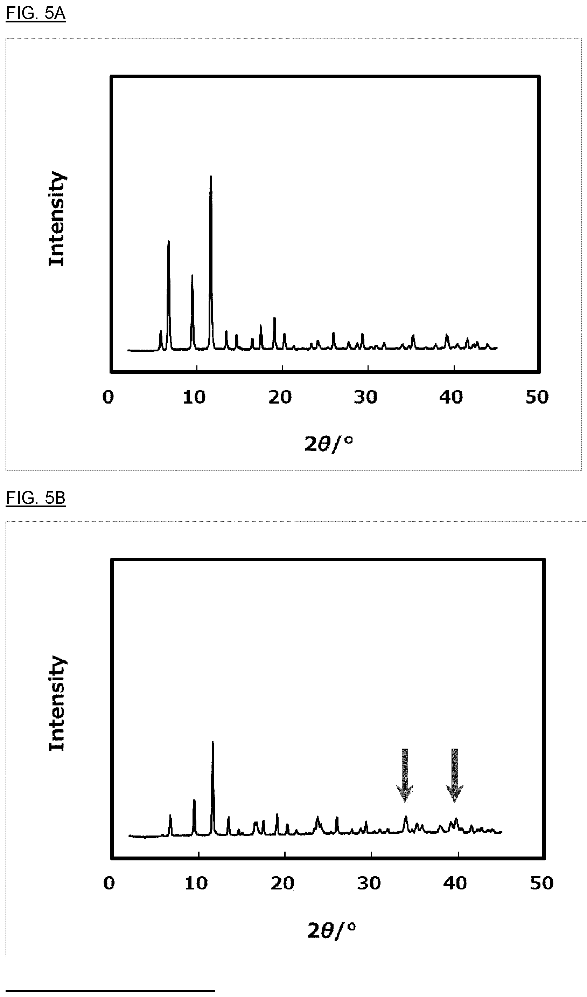

FIG. 5A shows the XRD pattern of the Metal-Organic Framework (HKUST-1) synthesized under the conditions of Example 1. FIG. 5B shows the XRD pattern of the Metal-Organic Framework (HKUST-1) synthesized under the conditions of Comparative Example A2. As can be seen from a comparison of the two, in FIG. 5B, the peak of the raw material copper(II) hydroxide (indicated by two arrows) remains. From this result, it can be seen that when the homomixer was used, a Metal-Organic Framework (HKUST-1) of higher quality was obtained compared to when the stirrer was used. This difference is also reflected as a difference in BET specific surface area.

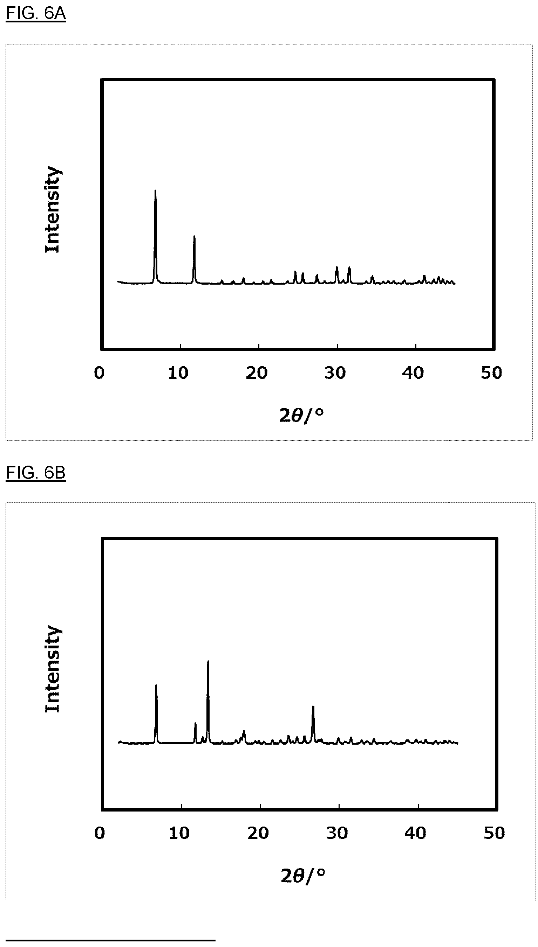

FIG. 6A shows the XRD pattern of the Metal-Organic Framework [MOF-74(Mg)] synthesized under the conditions of Example 7. FIG. 6B shows the XRD pattern of the Metal-Organic Framework [MOF-74(Mg)] synthesized under the conditions of Comparative Example A4. As can be seen from the comparison of the two, when the synthesis is performed on a large scale (about 2 kg of metal ion donor by weight), high-quality Metal-Organic Frameworks are obtained under the former conditions, whereas under the latter conditions, the Metal-Organic Frameworks are not successfully synthesized. This difference is also reflected as a difference in BET specific surface area.

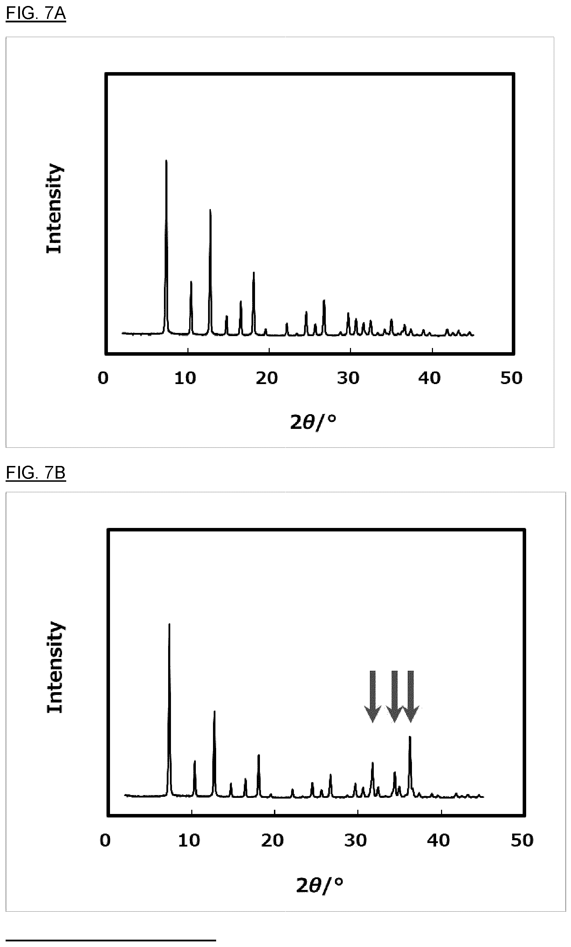

FIG. 7A shows the XRD pattern of the Metal-Organic Framework (ZIF-8) synthesized under the conditions of Example 12. FIG. 7B shows the XRD pattern of the Metal-Organic Framework (ZIF-8) synthesized under the conditions of Comparative Example A13. As can be seen from a comparison of the two, the peaks of zinc oxide, the raw material, remain in FIG. 7B (indicated by three arrows). From this result, it can be seen that when the homomixer was used, a Metal-Organic Framework (ZIF-8) of higher quality was obtained compared to when the stirrer was used. This difference is also reflected as a difference in BET specific surface area.

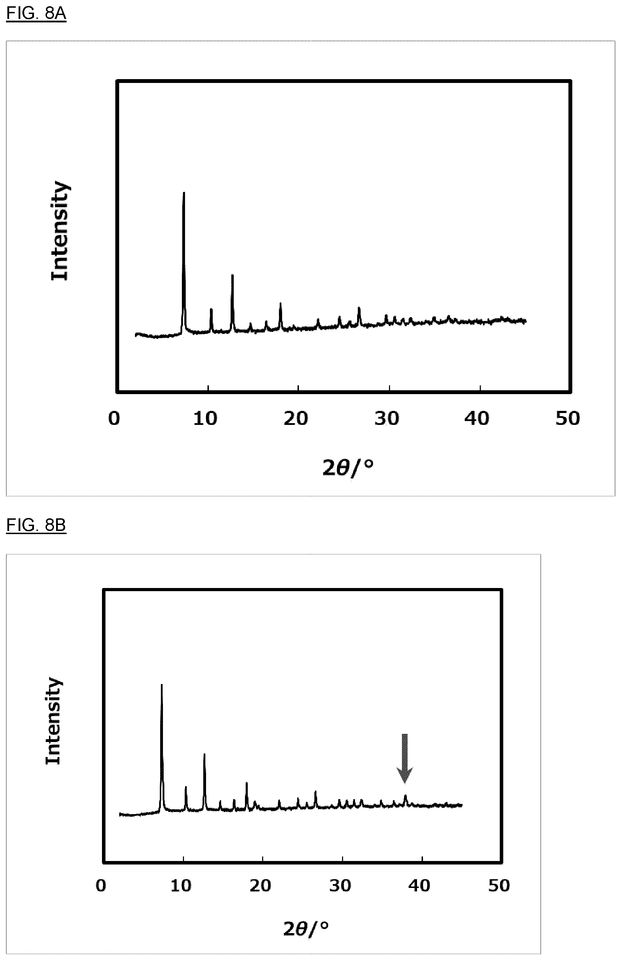

FIG. 8A shows the XRD pattern of the Metal-Organic Framework (ZIF-67) synthesized under the conditions of Example 26. FIG. 8B shows the XRD pattern of the Metal-Organic Framework (ZIF-67) synthesized under the conditions of Comparative Example A32. As can be seen from a comparison of the two, in FIG. 8B, the peak of the raw material cobalt (II) hydroxide (indicated by a single arrow) remains. From this result, it can be seen that when the homomixer was used, a Metal-Organic Framework (ZIF-67) of higher quality was obtained compared to when the homodisper was used. This difference is also reflected as a difference in BET specific surface area.

As described above, it has been found that by using a turbine/stator-type high-speed agitator, Metal-Organic Frameworks of higher quality can be synthesized while significantly reducing the reaction time, compared to using high-speed stirring blades or a stirrer, for a wide variety of Metal-Organic Frameworks. Similarly, it has been found that by using a turbine/stator-type high-speed agitator, Metal-Organic Frameworks of equal or better quality can be synthesized at a larger scale, compared to using a swirling thin-film type high-speed agitator, for a wide variety of Metal-Organic Frameworks.

## Claims

1. A method for producing a Metal-Organic Framework, comprising:
preparing an agitator equipped with a turbine and a stator surrounding the turbine;
preparing a formulation comprising a metal ion donor, a multidentate ligand, and a solvent; and
applying shear forces generated between the turbine and the stator to the formulation by rotating the turbine relative to the stator in the agitator.

2. The method according to claim 1, wherein the solvent is a poor solvent for at least one of the metal ion donor and the multidentate ligand.

3. The method according to claim 1 or 2, wherein the solvent has a Hildebrand solubility parameter of 9.0 or more.

4. The method according to claim 1 or 2, wherein an amount of the solvent is in a range of 25 to 2000% by weight based on a total amount of the metal ion donor and the multidentate ligand.

5. The method according to claim 1 or 2, wherein the method is carried out at a temperature lower than a normal boiling point of the solvent.

6. The method according to claim 1 or 2, wherein the shortest distance between the turbine and the stator is in the range of 0.1 mm to 5 mm.

7. The method according to claim 1 or 2, wherein the Metal-Organic Framework is a Porous Coordination Polymer.
